# EUROPEAN PATENT SPECIFICATION

(11) **EP 0 699 443 B1**
(45) Date of publication and mention of the grant of the patent: **03.11.1999**
(21) Application number: 95107589.4
(22) Date of filing: 18.05.1995
(51) Int. Cl.: A61K 35/78, A61K 7/48

(54) **Elder-containing skin preparation**
Holunder enthaltendes Hautpflegemittel
Composition pour la peau contenant du sureau

(30) Priority: 02.08.1994 IT MI941680
(43) Date of publication of application: 06.03.1996
(73) Proprietor: Melis, Luigi, I-07030 Laerru (Sassari) (IT); Solinas, Mario, I-07100 Sassari (IT)
(72) Inventor: Melis, Luigi, I-07030 Laerru (Sassari) (IT); Solinas, Mario, I-07100 Sassari (IT)
(74) Representative: Sama, Daniele, Dr.

(56) References cited:
- AT-B- 393 451
- FR-M- 696
- DATABASE WPI Week 9438 Derwent Publications Ltd., London, GB; AN 94-304834 & ES-A-2 056 029 (IGLESIAS VITORES R M) , 16 September 1994
- DATABASE WPI Week 9429 Derwent Publications Ltd., London, GB; AN 94-236928 & ES-A-2 052 443 (VIDUEIRA GARCIA M) , 1 July 1994
- PATENT ABSTRACTS OF JAPAN vol. 18 no. 427 (C-1235) ,10 August 1994 & JP-A-06 128145 (KOSE CORP) 10 May 1994,

## Description

The present invention relates to vegetable extracts generally for skin lesions, in particular for burns, sores and solar erythemas.

It is known that many lesions are treated without turning to the doctor, such as for instance those caused by small wounds and burns. They are very frequent lesions and need a simple but immediate treatment. The skin in fact is a protective coating which acts as a first physical barrier to the penetration of germs present in the environment.

When the skin is damaged owing to lesions, said barrier is temporarily lost until the wound heals. A quick treatment of the lesion reduces the risk of infection and allows a quicker recovery.
Several products for healing ulcerative lesions caused by decubitus ulcers, burns caused by boiling water, acids or solvents in general, red-hot irons, are known in trade, which however show poor practical results. For instance the decubitus ulcers generally appear in the patient confined to bed, in particular in patients compelled to maintain the sitting position for a long time.

20% of patients (over 65 years old), develops decubitus ulcers during stay in hospital.

An incidence of sores equal to about 24% in homes has been reported in Italy. The phenomenon is however general for this kind of patients.

The cost of medical and nursing assistance for these patients is very high. Moreover the decubitus ulcers increase the mortality in geriatric patients. It is necessary, moreover, to point out the stress and the troubles both for patients and for people looking after them.

The products normally used in trade for first degree burns are cortison-based creams or antihistamines, for second degree burns associated antibiotics and cortison-based products.

The drawbacks of cortison-based creams consist in that they cannot be used for prolonged periods both because their effectiveness decreases as they are used, and because when the treatment is stopped, the lesions reappear.

If itch is present, cortison-based treatments increase it.

Other products known in trade that can be mentioned are placenta creams, based on placenta extracts commercialized by Geymonat, retinol-based Euvitol creams of Smithkline Beecham (vitamin A), Trofodermin of Carlo Erba based on clobestol and neomycin.

These products are utilized as cicatrizants and each of them has particular characteristics which settle their use in specific cases. They can cause allergic phenomena in the application zone with irritation of the skin and itches.

There are also antibiotic salves which however do not give satisfactory results.

Elder tree medical properties are known in the art and several parts of this plant are used for the treatment of numerous pathologies.

ES-A-2052443 deals with a cream in which a 5% of grating pulp of elderberry bark endodermis is contained together with 35% of tallow or saturated animal fat, 35% of unrefined virgin olive oil and 25% of virgin wax. Such a cream is used for the treatment of skin burns.

FR-M-696 concern a medicament obtained from the leaves of Sambucus Ebulus in the form of powder or alcoholic extract in combination with any dermathologic means (such salve or cream). Said medicament is used for treating sores and burns.

AT-B-393451 deals with a medicament obtained from flowers of Sambucus nigra combined with dimethylaminobenzoic acid. Said extract is used for treating solar erythemas and as UV protecting agent.

Therefore the need was felt of a new kind of medicament allowing to obtain first of all an immediate relief, i.e. a feeling of freshness, combined with a remarkable effectiveness within a limited time and restoration of the epidermis without leaving any healing.

It has been unexpectedly found that if an extract of vegetable origin is utilized, as defined hereafter, one succeeds in overcoming the drawbacks of the known products and moreover it combines a feeling of immediate relief with a healing of the ulcerative lesions or burns in short times and restoring the epidermis at the previous state without any healing of the skin.

Object of the present invention is therefore the use of an elder decoction for preparing a medicament for the lesions treatment, in particular burns, sores, solar erythemas, on the human or animal body, where said decoction is obtained from elder branches, preferably two-years-old branches, submitted to boiling in water for a time generally from 15' to 10 hours, preferably for times from 1 to 2 hours, then it is cooled and the decoction is recovered.

The amount of branches generally ranges from 100 to 300 g for liter of water.

The decoction keeps very well, preferably in refrigerator and in the absence of light.

Without binding to any action mechanism, it is assumed that the decoction has an action of tissular regeneration since capable of restoring all the alterations of the dermic tissue due to burns or to decubitus ulcers without leaving healing. It is useful that the treated skin surface is not covered by gauzes or the like, since the contact with air favours the decoction effectiveness. Moreover it has been surprisingly noticed that the skin hairs normally grow again in the burnt parts.

Tests carried out by the Applicants have shown that the action mechanism of the decoction is independent from the kind of the burn degree.

As already said, an example of another application of the decoction of the present invention is that of the solar erythemas treatment. For this use an immediate feeling of freshness is stressed in patients, stopping the strong burning characteristic of burns and skin irritations.

Another application is for abrasions, excoriations, etc.

The dosage utilized ranges from 2/3 applications a day for a period of about 15 days for simple lesions to 30/60 days for the more serious lesions.

Various experiments carried out by the Applicants have shown that the effective part of the elder for the lesions treatment is the extract obtained from the bark, while the flowers and the berries of this plant do not result to have any meaningful effect.

The effectiveness of the lesions treatment is much more surprising as the elder flowers of the invention applied on the skin cause strong irritation with erythema.

The elder utilized to obtain the decoction of the present invention is known under the name of Sambucus nigra L. (Caprifoliaceae) according to what reported in the most common reviews on medicinal plants. It is generally found in moist woods and also in the highlands of South Europe, West Asia, North Africa, Azores and at heights generally varying from 0 to 1,400 m. In Italy it can be found in Sardinia in the places indicated in the mentioned review. It is easily found in the valley streets and in their groves periodically flooded: in Italy it is frequent in hedges and in ruins, near villages, less frequent in woods and along watercourses, from plain to subalpine zones, in the Peninsula, Sicily, Sardinia.

According to the present invention the part utilizable for obtaining the decoction is formed by the bark. The more important constituents of the bark are: tannin, sambucine, laxative resin, essence, sambucigrine, potassium nitrate, sugars and two substances C₂₇H₄₈O and C₂₃H₄₀O₂.

The decoction utilizable according to the present invenzion has concentration normally of 5/10% by weight. In general the bark and branches, before being utilized, must be well dried so that they loose the toxic components contained therein. The decoction can be applied by spray or by simple spreading, also by brush.

The following examples are given for illustrative purpose but not limitative of the present invention.

### EXAMPLE 1 (comparative): decubitus ulcers

A patient, 82 years-old, discharged from hospital showed decubitus ulcers concerning the sacral zone and heels.

The ulcerative lesions are of a certain seriousness, some concern the superficial part of the skin, others the derma and the subskin, with rounded skin edges.

A classic method was utilized for healing sores.

The skin was kept clean and dry, changing the patient position in bed every 2/3 hours.

After accurate washings with physiologic solution, a polyurethane medication was applied.

Said medication being permeable only to gas and to steam, hinders liquids from infecting the wound.

After seven days of treatment no result was obtained. Even continuing the treatment for 15 days, no appreciable result occurred.

### ESAMPLE 2: decubitus ulcers

To the same patient of Example 1 the vegetable composition according to the present invention, elder decoction, was applied.

The following method was used: the ulcer was cleaned with physiologic solution and then the aforesaid decoction was sprayed on the ulcer.

The treatment is initially carried out three times a day, then twice a day.
The patient had an immediate benefit feeling an wellbeing sensation. After four days of treatment a healing of the lesion was noticed with reduction of the ulcer size.

For the recovery of the II degree ulcers 15 days of treatment were necessary, at the frequencies indicated above; for those of III degree 30 days of treatment were sufficient.

### EXAMPLE 3: burns

A patient, 45 years old, showed burns caused by boiling water.

The burns concerned the face, upper limbs, the posterior trunk and were of II and III degree and had an extension of about 30% of the surface of the indicated parts.

The patient was submitted to a treatment with the elder decoction according to the following method.

The decoction was sprayed on the ulcers, taking care of removing the blisters roof, to treat the underlying lesion.

The medication was carried out three times/die for the first 10 days, then twice/die. After three days a little crust formed on the burn, which was humidified with vegetable oil. After a treatment of 25 days the recovery of the wounds took place without any scar on the skin, recovering the epidermis at the state as it was prior to the burn.

### EXAMPLE 4: burns

A patient, 30 years old, showed burns from sulphuric acid concerning the face.

The treatment with the elder decoction was carried out according to the modalities described in Example 3. After 25 days of application the recovery of the wounds took place without any scar on the skin, recovering the epidermis at the state as it was prior to the burn.

## Claims

1. Decoction of Sambucus nigra L. of the Caprifoliaceae family for preparing a medicament for the lesions treatment on the human or animal body characterized in that two years old branches are submitted to boiling in water from a time generally from 15' to 10 hours, then it is cooled and the decoction is recovered.

2. Use of the decoction of Sambucus nigra L. of the Caprifoliaceae family according to Claim 1 as a medicament for decubitus ulcer lesions treatment on the human or animal body.

3. Use of the decoction of Sambucus nigra L. of the Caprifoliaceae family according to Claim 1, wherein the lesions are caused by burns and solar erythemas.

4. Use according to Claims 2 and 3, wherein the decoction is applied to the lesions by simple spreading or by brush.

## Patentansprüche

1. Dekokt von Sambucus nigra L. aus der Familie der Caprifoliaceae zur Herstellung eines Arzneimittels für die Behandlung von Läsionen am menschlichen oder tierischen Körper,
**dadurch gekennzeichnet, daß**
zwei Jahre alte Zweige einem Kochen in Wasser für eine Zeitdauer von im wesentlichen 15 Minuten bis 10 Stunden unterzogen werden, dann eine Abkühlung stattfindet und das Dekokt gewonnen wird.

2. Verwendung des Dekokts von Sambucus nigra L. aus der Familie der Caprifoliaceae gemäß Anspruch 1 als ein Arzneimittel zur Behandlung von Dekubitalulkus-Läsionen am menschlichen oder tierischen Körper.

3. Verwendung des Dekokts von Sambucus nigra L. aus der Familie der Caprifoliaceae gemäß Anspruch 1, wobei die Läsionen durch Verbrennungen und Sonnenerytheme verursacht sind.

4. Verwendung gemäß einem der Ansprüche 2 und 3, wobei das Dekokt durch einfaches Verteilen oder mittels eines Pinsels auf die Läsionen aufgebracht wird.

## Revendications

1. Décoction de *Sambucus nigra L.* de la famille des Caprifoliacées pour. préparer un médicament destiné au traitement des lésions du corps humain ou du corps des animaux, caractérisée en ce qu'on fait bouillir des branches de deux aus dans l'eau pendant une durée généralement comprise entre 15 minutes et 10 heures. ensuite on refroidit et on récupère la décoction.

2. Utilisation de la décoction de *Sambucus nigra L*. de la famille des Caprifoliacées selon la revendication 1, comme médicament pour le traitement des lésions ulcéreuses du décubitus sur les patients humains ou les animaux.

3. Utilisation de la décoction de *Sambucus nigra L.* de la famille des Caprifoliacées selon la revendication 1, dans laquelle les lésions sont occasionnées par des brûlures et des érythèmes solaires.

4. Utilisation selon la revendication 2 et 3, dans laquelle la décoction est appliquée sur les lésions par simple étalement ou au pinceau.
